# EUROPEAN PATENT APPLICATION

(11) **EP 3 447 718 A1**
(43) Date of publication of application: **27.02.2019**
(21) Application number: 17785798.4
(22) Date of filing: 05.04.2017
(51) Int. Cl.: G06Q 50/24, A61J 3/00, G06F 17/30

(54) **SUBSTITUTE MEDICINE DETECTION DEVICE AND SUBSTITUTE MEDICINE DETECTION METHOD**

(30) Priority: 18.04.2016 JP 2016082954
(71) Applicant: FUJIFILM Corporation, Tokyo 106-8620 (JP)
(72) Inventor: HASEGAWA, Kazuhide, Ashigarakami-gun Kanagawa 258-8538 (JP)
(74) Representative: Klunker IP Patentanwälte PartG mbB
(86) International application number: PCT/JP2017/014259
(87) International publication number: WO 2017/183456

(57) **Abstract**

Provided are an alternative medicine retrieval apparatus and an alternative medicine retrieval method capable of presenting information regarding an alternative medicine sufficiently suitable for a user's preference and a pharmacy handling the alternative medicine to the user. An alternative medicine retrieval apparatus includes a database (30) that stores medicine information including attribute information of a medicine, a prescription slip data acquisition unit (50) that acquires prescription slip data, a preference information acquisition unit (52) that acquires preference information of a user regarding the attribute information of the medicine, a retrieval unit (54) that retrieves an alternative medicine by referring to the prescription slip data, the medicine information, and the preference information of the user, an information presenting unit that presents, to the user, alternative medicine information and information regarding a pharmacy handling a prescribed medicine and a pharmacy handling the retrieved alternative medicine, and an inspection information acquisition unit (42) that acquires medicine inspection information in the pharmacies, in which the database (30) updates the attribute information according to acquisition of the medicine inspection information.

## Description

### BACKGROUND OF THE INVENTION

### 1. Field of the Invention

The present invention relates to an alternative medicine retrieval apparatus and an alternative medicine retrieval method capable of presenting information regarding an alternative medicine sufficiently suitable for a user's preference and a pharmacy handling the alternative medicine to the user.

### 2. Description of the Related Art

There is a case where a medicine (hereinafter, referred to as an "alternative medicine") which can replace a prescribed medicine is purchased in a pharmacy. For example, a prescribed medicine is an original medicine, and a generic medicine having the same active ingredient as that of the original medicine is purchased as an alternative medicine. The generic medicine is sold after validity and safety are recognized in the same manner as the original medicine. Such a generic medicine can be made cheaper than an original medicine by lower development cost. In contrast with an original medicine, a generic medicine may be improved and sold in a form of being easily taken, through a study on reducing a tablet size or coating a tablet.

JP2012-203466A discloses a pharmacy retrieval system which examines an inventory of alternative medicines having the same effect as that of a prescribed medicine for each pharmacy, and presents pharmacies capable of selling the alternative medicine.

JP2006-502814A discloses an integrated pharmacy system in which a substitute rule for a medicine is stored in advance, and a medicine suitable for a customer is selected from a list of prescribed medicines and alternative medicines on the basis of the substitute rule. The substitute rule includes a rule based on a customer's preference such as whether the customer's favorite is a generic medicine or a named medicine.

JP2003-196394A discloses a prescription determination system in which, in a case where a patient name and a drug name are entered, a main ingredient and the suitability of a taking form of a drug for a patient are determined on the basis of personal information and drug information of the patient stored in advance. As the personal information, not only fixed information such as the sex of the patient, the patient's date of birth, and an insurance number but also information such as a symptom which changes over time is entered by a doctor or a pharmacist. For example, a patient having difficulty in taking a drug (a capsule or a tablet) in a swallowing form or a patient having difficulty in taking a powdered drug, such as a patient of which a swallowing ability is reduced or a patient having a throat disease, is registered in advance as personal information in a preferable taking form for each patient, and the suitability is determined for each patient.

JP2003-141252A discloses a prescription information service system in which information excluding information for specifying a patient is extracted from information written on a prescription slip, so that prescription result data is created for each medicine, and the prescription result data is provided to a pharmaceutical company.

### SUMMARY OF THE INVENTION

However, it is practically hard to present information regarding an alternative medicine sufficiently suitable for a user's preference and a pharmacy handling the alternative medicine to the user.

In the technique disclosed in JP2012-203466A, a pharmacy having an inventory of alternative medicines is merely examined, and is presented to a user, and thus it cannot be said that information regarding a medicine suitable for a user's preference and a pharmacy handling the alternative medicine is presented to the user.

In the technique disclosed in JP2006-502814A, to summarize, one of a prescribed medicine and an alternative medicine is merely selected on the basis of whether a customer's preference such as whether the customer's favorite is a generic medicine or a named medicine, and thus it cannot be said that information regarding an alternative medicine sufficiently suitable for a user's preference and a pharmacy handling the alternative medicine is presented to the user.

In the technique disclosed in JP2003-196394A, to summarize, since personal information including a medicine taking form (a capsule, a tablet, powder, or the like) is required to be registered in advance for each patient, and information entry depends on a doctor or a pharmacist, even in a case where this is applicable to retrieval of an alternative medicine, it is practically hard to present information regarding an alternative medicine sufficiently suitable for a patient's preference and a pharmacy handling the alternative medicine. This is because, first, work of registering attribute information of a medicine requires considerable time and effort. In other words, various pieces of attribute information of a medicine are required to be registered in advance in order to be able to retrieve an alternative medicine sufficiently suitable for a patient's preference, but complicated registration work causes a great burden. Second, doctors and pharmacists are very limited human resources. In other words, a doctor and a pharmacist having abundant knowledge about a patient's symptom, a medicine, and relevance therebetween can enter preferable attribute information for each patient, but it is practically hard for busy doctors and pharmacists to perform registration work.

According to the technique disclosed in JP2003-141252A, information written on a prescription slip can be collected, but attribute information not written on the prescription slip cannot be collected.

Therefore, even in a configuration disclosed in any of JP2012-203466A, JP2006-502814A, JP2003-196394A, and JP2003-141252A, or a configuration obtained by combining the configurations disclosed in JP2012-203466A, JP2006-502814A, JP2003-196394A, and JP2003-141252A, it can be said that it is hard to present information regarding an alternative medicine sufficiently suitable for a user's preference and a pharmacy handling the alternative medicine to the user.

The present invention has been made in consideration of these circumstances, and an object thereof is to provide an alternative medicine retrieval apparatus and an alternative medicine retrieval method capable of presenting information regarding an alternative medicine sufficiently suitable for a user's preference and a pharmacy handling the alternative medicine to the user.

In order to achieve the above-described object, according to a first aspect of the present invention, there is provided an alternative medicine retrieval apparatus including a medicine information storage unit that stores medicine information including identification information of a medicine, attribute information of the medicine, and identification information of a medicine which is able to replace the medicine; a preference information acquisition unit that acquires preference information of a user regarding the attribute information; a prescription slip data acquisition unit that acquires prescription slip data of a first user; a pharmacy information acquisition unit that acquires pharmacy information including information regarding a medicine handled in a pharmacy and inventory information of the handled medicine; a retrieval unit that retrieves an alternative medicine which is able to replace a prescribed medicine designated in the prescription slip data, and retrieves the alternative medicine by referring to the prescription slip data, the medicine information, and preference information of the first user in response to a request from the first user; an information presenting unit that presents alternative medicine information including a result of the retrieval, and the pharmacy information regarding a pharmacy handling the prescribed medicine and a pharmacy handling the retrieved alternative medicine, to the first user; and an inspection information acquisition unit that acquires medicine inspection information in the pharmacies, in which the medicine information storage unit updates the attribute information according to acquisition of the medicine inspection information.

According to this aspect, since an alternative medicine suitable for preference information of a user is retrieved by referring to medicine attribute information which is updated and accumulated in the pharmacy information storage unit according to acquisition of medicine inspection information, in a case where fundamental attribute information (for example, a taking form) has only to be registered in advance for each medicine, or even in a case where there is a medicine of which fundamental attribute information is not registered, an alternative medicine is retrieved on the basis of attribute information which is updated and accumulated according to acquisition of medicine inspection information. In other words, according to this aspect, it is possible to present information regarding an alternative medicine sufficiently suitable for a user's preference and a pharmacy handling the alternative medicine to the user.

The alternative medicine retrieval apparatus according to the second aspect of the present invention further includes a pharmacy information storage unit that stores the pharmacy information, and the pharmacy information storage unit updates the pharmacy information according to acquisition of the medicine inspection information. According to this aspect, the pharmacy information accumulated in the pharmacy information storage unit is presented to the user according to acquisition of the medicine inspection information.

The alternative medicine retrieval apparatus according to a third aspect of the present invention further comprises a preference information storage unit that stores the preference information in correlation with replacement of the prescribed medicine with the alternative medicine; and a statistical processing unit that performs a statistical process on information stored in the preference information storage unit so as to generate statistical information. According to this aspect, preference information is collected and is subjected to a statistical process, and statistical information useful for manufacturing an alternative medicine can be provided to an alternative medicine manufacturer. It is possible to provide information regarding an alternative medicine favored by a user to a pharmacy.

In the alternative medicine retrieval apparatus according to a fourth aspect of the present invention, the preference information storage unit updates the preference information according to acquisition of the medicine inspection information. According to this aspect, it is possible to obtain statistical information with high reliability based on the medicine inspection information.

In the alternative medicine retrieval apparatus according to a fifth aspect of the present invention, the retrieval unit retrieves the alternative medicine by also referring to preference information of users other than the first user. According to this aspect, information regarding an alternative medicine and a handling pharmacy retrieved by also referring to preference information of other users who are different from the first user having requested the retrieval is presented to the first user. For example, an alternative medicine can be retrieved with preference information based on collective statistics as a retrieval key, and information regarding the alternative medicine and a handling pharmacy can be presented to the first user who is a retrieval request source.

In the alternative medicine retrieval apparatus according to a sixth aspect of the present invention, the alternative medicine information includes preference information of users other than the first user. According to this aspect, since preference information of users other than the first user is also presented to the first user, the first user can determine whether or not the presented alternative medicine can be purchased by also referring to the preference information of users other than the first user.

In the alternative medicine retrieval apparatus according to a seventh aspect of the present invention, in a case where the preference information of the first user is different from preference information of users other than the first user, the information presenting unit prioritizes the preference information of the first user, and presents the alternative medicine information. For example, a first alternative medicine which is retrieved with personal preference information of the first user having requested the retrieval as a retrieval key can be displayed and output with higher visibility than that of a second alternative medicine which is retrieved with preference information based on collective statistics as a retrieval key.

The alternative medicine retrieval apparatus according to an eighth aspect of the present invention further includes a determination unit that determines a suitability of the alternative medicine for the preference information of the first user, and the information presenting unit presents information regarding the retrieved alternative medicine to the first user along with the determined suitability. According to this aspect, since the suitability of an alternative medicine for preference information of the first user is presented to the first user, the first user can easily determine whether or not the presented alternative medicine is purchased by referring to the suitability.

In the alternative medicine retrieval apparatus according to a ninth aspect of the present invention, the inspection information acquisition unit acquires an image obtained by imaging the medicine as the medicine inspection information, and the medicine information storage unit updates the attribute information on the basis of the image. According to this aspect, attribute information is updated on the basis of a medicine image obtained by imaging a medicine, and thus the attribute information can be easily updated.

In the alternative medicine retrieval apparatus according to a tenth aspect of the present invention, the attribute information includes first attribute information which is attribute information understood objectively and second attribute information which is attribute information understood subjectively by the user.

In the alternative medicine retrieval apparatus according to an eleventh aspect of the present invention, the preference information includes first preference information which is preference information corresponding to the first attribute information and second preference information which is preference information corresponding to the second attribute information. According to this aspect, an alternative medicine can be retrieved on the basis of the first preference information for the first attribute information understood objectively, so as to be presented to a user, and an alternative medicine can also be retrieved on the basis of the second preference information for the second attribute information understood subjectively by the user, so as to be presented to the user.

In the alternative medicine retrieval apparatus according to a twelfth aspect of the present invention, the second attribute information is sensitivity words expressing sensitivity of a person corresponding to the first attribute information, the medicine information storage unit stores conversion information indicating a correspondence relationship between the first attribute information and the second attribute information, and, in a case where the second preference information is acquired by the preference information acquisition unit, the retrieval unit coverts the second preference information into the first preference information on the basis of the conversion information, and retrieves the alternative medicine on the basis of the first preference information. According to this aspect, even in a case where the second attribute information understood subjectively by a user is not correlated with identification information of a medicine, as long as the first attribute information understood objectively is correlated with the identification information of the medicine, an alternative medicine can be retrieved with the second attribute information understood subjectively by the user as a retrieval key, and information regarding the alternative medicine and a handling pharmacy can be presented to the user.

In the alternative medicine retrieval apparatus according to a thirteenth aspect of the present invention, the preference information of the first user is updated according to a result of the retrieval. According to this aspect, preference information of a user is updated according to a retrieval result (typically, a content presented to the user).

The alternative medicine retrieval apparatus according to a fourteenth aspect of the present invention further includes an order reception unit that receives an order for medicine purchase corresponding to a result of the retrieval from the first user, and the preference information of the first user is updated according to a content of the order. According to this aspect, the preference information of the first user is updated according to a content of the order for medicine purchase from the first user, and an alternative medicine suitable for the updated preference information of the first user is retrieved to be presented to the user.

In the alternative medicine retrieval apparatus according to a fifteenth aspect of the present invention, the prescription slip data acquisition unit acquires the prescription slip data on the basis of a prescription slip image obtained by imaging a prescription slip. According to this aspect, a prescribed medicine can be specified on the basis of a prescription slip image obtained by imaging a prescription slip, and an alternative medicine for the prescribed medicine can be retrieved to be presented to the user.

In the alternative medicine retrieval apparatus according to a sixteenth aspect of the present invention, the preference information acquisition unit acquires information regarding a medicine which the user has experience of taking, as the preference information. According to this aspect, it is possible to present an alternative medicine corresponding to a medicine which a user has experience of taking and a handling pharmacy to the user.

In the alternative medicine retrieval apparatus according to a seventeenth aspect of the present invention, the preference information acquisition unit requests the user to present the preference information, and acquires, as the preference information, information presented by the user in response to the request. According to this aspect, a user can be requested to present preference information, and an alternative medicine suitable for information which is presented by the user in response to the request can be retrieved to be presented to the user.

According to an eighteenth aspect of the present invention, there is provided an alternative medicine retrieval method including a medicine information acquisition process of acquiring medicine information including identification information of a medicine, attribute information of the medicine, and identification information of a medicine which is able to replace the medicine; a pharmacy information acquisition process of acquiring pharmacy information including information regarding a medicine handled in a pharmacy and inventory information of the handled medicine; a preference information acquisition process of acquiring preference information of a user regarding the attribute information; a prescription slip data acquisition process of acquiring prescription slip data of a first user; a retrieval process of retrieving an alternative medicine which is able to replace a prescribed medicine designated in the prescription slip data, and retrieving the alternative medicine by referring to the prescription slip data, the medicine information, and preference information of the first user in response to a request from the first user; an information presenting process of presenting alternative medicine information including a result of the retrieval, and the pharmacy information regarding a pharmacy handling the prescribed medicine and a pharmacy handling the retrieved alternative medicine, to the first user; and an inspection information acquisition process of acquiring medicine inspection information in the pharmacies, in which, in the medicine information acquisition process, the attribute information is updated according to acquisition of the medicine inspection information.

According to the present invention, it is possible to present information regarding an alternative medicine sufficiently suitable for a user's preference and a pharmacy handling the alternative medicine to the user.

### BRIEF DESCRIPTION OF THE DRAWINGS

Fig. 1 is a system configuration diagram illustrating an example of a configuration of an alternative medicine retrieval system in a first embodiment.
Fig. 2 is an explanatory diagram for explaining a data configuration example of a database.
Fig. 3 is an explanatory diagram for explaining a medicine information example.
Fig. 4 is an explanatory diagram for explaining conversion information indicating a correspondence relationship between first attribute information which is objectively understood and second attribute information which is subjectively understood by a user.
Fig. 5 is an explanatory diagram for explaining a pharmacy information example.
Fig. 6 is an explanatory diagram for explaining a preference information example.
Fig. 7 is a block diagram illustrating a configuration example of a server apparatus in the first embodiment.
Fig. 8 is a flowchart illustrating a flow of an alternative medicine retrieval process example.
Fig. 9 is an explanatory diagram for explaining an example of presentation to a user.
Fig. 10 is an explanatory diagram for explaining update of medicine information.
Fig. 11 is an explanatory diagram for explaining update of pharmacy information.
Fig. 12 is an explanatory diagram for explaining update of preference information.
Fig. 13 is an explanatory diagram for explaining a statistical process.
Fig. 14 is a block diagram illustrating a configuration example of an alternative medicine retrieval system in a second embodiment.
Fig. 15 is a block diagram illustrating a configuration example of a pharmacy apparatus configuring an alternative medicine retrieval apparatus in the second embodiment.

### DESCRIPTION OF THE PREFERRED EMBODIMENTS

Hereinafter, with reference to the drawings, a description will be made of an alternative medicine retrieval apparatus and an alternative medicine retrieval method according to embodiments of the present invention.

### First Embodiment

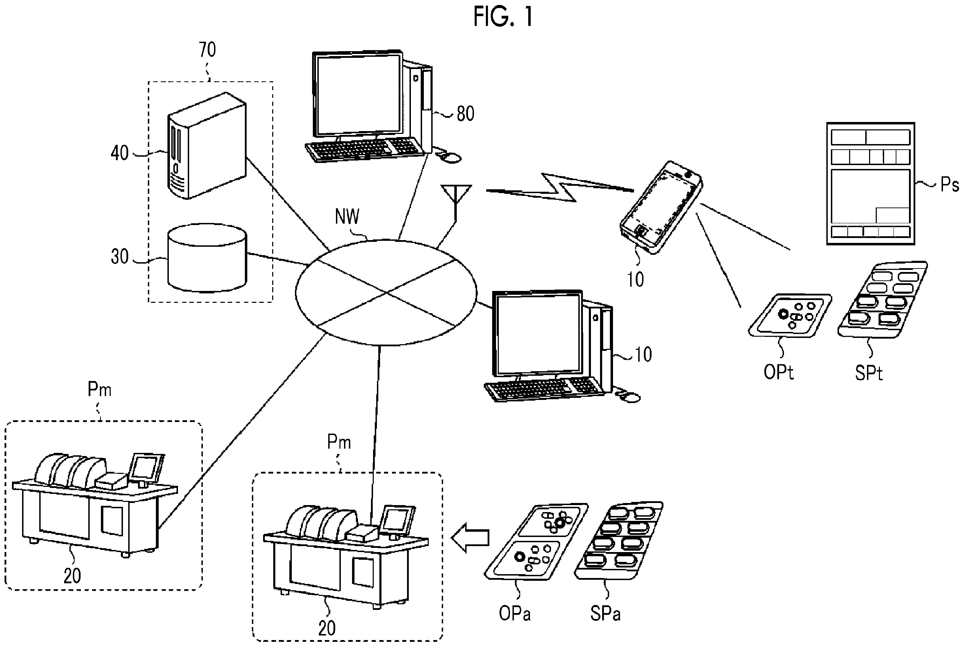

Fig. 1 is a system configuration diagram illustrating an example of a configuration of an alternative medicine retrieval system in a first embodiment.

In Fig. 1, an alternative medicine retrieval system is configured to include a user terminal 10, an inspection apparatus 20, a database 30 (an aspect of an "medicine information storage unit", a "pharmacy information storage unit", and "preference information storage unit"), a server apparatus 40, and an administrator terminal 80. In this example, an alternative medicine retrieval apparatus 70 is configured with the database 30 and the server apparatus 40. The user terminal 10, the inspection apparatus 20, the database 30, the server apparatus 40, and the administrator terminal 80 are connected to a network NW.

The user terminal 10 is configured with a computer apparatus. For example, the user terminal 10 is configured with a smart phone or a personal computer.

The inspection apparatus 20 is disposed in each of a plurality of pharmacies Pm, and performs an inspection of collating a medicine (hereinafter, referred to as a "prescribed medicine") designated in a prescription slip Ps with an inspection target medicine (for example, one-dose package drugs OPa or sheet package drugs SPa) actually prepared. In the figure, the one-dose package drugs OPa are a packaged medicine containing a plurality of types of drugs in one package every dose. In Fig. 1, the sheet package drugs SPa is a packaged medicine (for example, a press through package (PTP) sheet) containing a plurality of drugs of the same type of multiple doses in one sheet. The inspection apparatus 20 in this example performs an inspection of collating a medicine (hereinafter, referred to as an "alternative medicine") which is not directly designated in the prescription slip Ps but has the same active ingredient as a prescribed medicine and can thus replace a prescribed medicine with an actually prepared medicine which is an inspection target.

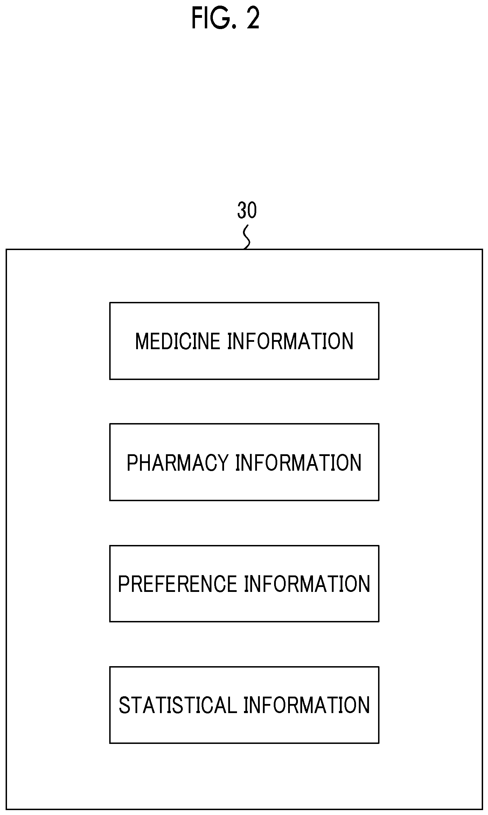

As illustrated in Fig. 2, the database 30 stores medicine information regarding each of a plurality of medicines, pharmacy information regarding each of a plurality of pharmacies, preference information regarding a preference of each of a plurality of users, and statistical information indicating a result of statistical processes on preference information of a plurality of users.

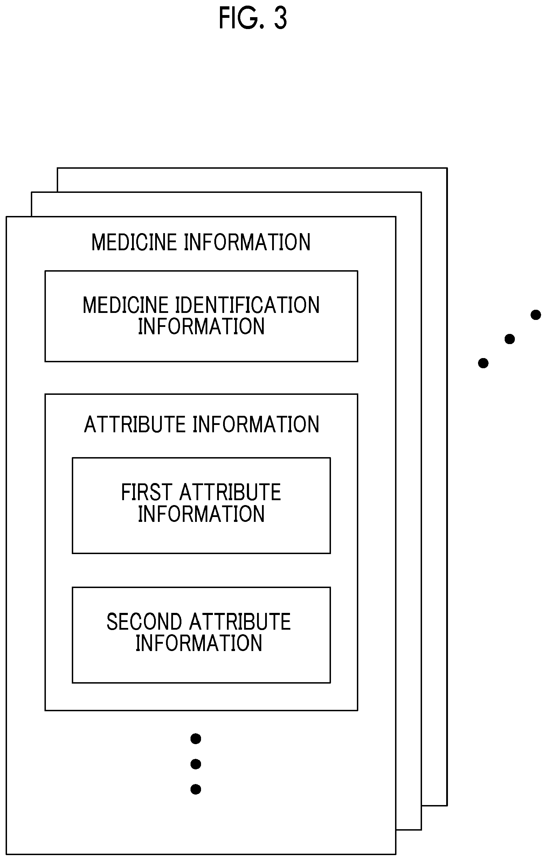

As illustrated in Fig. 3, the medicine information includes at least identification information of each medicine and attribute information of each medicine. A medicine includes a prescribed medicine and an alternative medicine which can replace the prescribed medicine. The identification information of a medicine includes identification information of a prescribed medicine and identification information of an alternative medicine. The attribute information of a medicine includes attribute information of a prescribed medicine and attribute information of an alternative medicine. In other words, the medicine information includes identification information of a prescribed medicine, attribute information of a prescribed medicine, identification information of an alternative medicine, and attribute information of an alternative medicine. The attribute information preferably includes manufacturer identification information (a company code) for identifying a manufacturer (pharmaceutical manufacturer) manufacturing a medicine.

The attribute information of a medicine includes first attribute information which is attribute information understood objectively and second attribute information which is attribute information understood subjectively by a user. The attribute information of the medicine information includes at least one of the first attribute information or the second attribute information.

The "attribute information understood objectively" is attribute information which is understood in common to users. A physical quantity (for example, a size, a shape, or a color of a medicine) and a chemical quantity (ingredients other than an active ingredient, for example, a coating ingredient) is the "attribute information understood objectively". The term "understood" includes a case where attribute information is understood on the basis of a printed or engraved content. Attribute information such as a taking form (a granule, a tablet, a capsule, no water, or the like), or the type of engraved mark is also objectively understood. The first attribute information includes information indicating at least one of a physical quantity of a medicine, a chemical quantity of a medicine, a printed content of a medicine, an engraved content of a medicine, or a medicine taking form.

The "attribute information understood subjectively" is attribute information understood for each user, and is attribute information which may be understood differently among users. First, the "attribute information understood subjectively" may be attribute information including sensitivity words representing the sensitivity of a person corresponding to the first attribute information, such as "easy to drink", "tastes good", "soft on the tongue", "easy to hold", or "easy to understand the medicine". Second, there is a user's opinion (evaluation) for each medicine, such as "highly effective" or "not effective". The "attribute information understood subjectively" may be a subjective opinion statistic. For example, the "attribute information understood subjectively" may be a statistic indicating effectiveness in collective opinions. The second attribute information includes information indicating at least one of sensitivity words, a user's opinion, or a statistic of users' opinions.

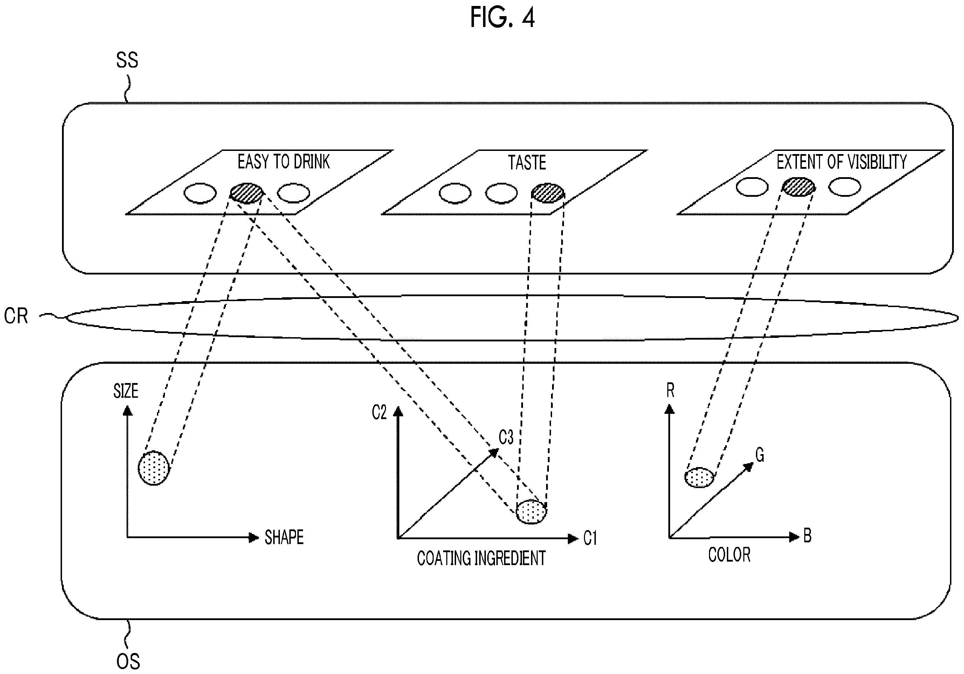

The database 30 in this example stores conversion information indicating a correspondence relationship between the first attribute information and the second attribute information. As illustrated in Fig. 4, a "size", a "shape", a "coating ingredient", a "color", and the like which are the first attribute information are included in an objective space OS. In Fig. 4, the "coating ingredient" is represented by three components (C1,C2,C3), and the color is represented by red (R), green (G), and blue (B), but these are only examples. "Drinkability" (for example, "easy to drink"), a "taste" (for example, "sweet"), the "extent of visibility" (for example, "easy to understand the medicine"), and the like which are the second attribute information represented by sensitivity words are included in a subjective space SS. The database 30 stores information (conversion information) indicating a correspondence relationship CR between the first attribute information in the objective space OS and the second attribute information in the subjective space SS. For example, a correspondence relationship between the "shape", the "size", and the "coating ingredient" in the objective space OS and the "drinkability" in the subjective space SS, a correspondence relationship between the "coating ingredient" in the objective space OS and the "taste" in the subjective space SS, and the "color" in the objective space OS and the "extent of visibility" in the subjective space SS are stored in the database 30 as conversion information. The first attribute information included in the objective space OS may be acquired from medicine inspection information which is an inspection result in the inspection apparatus 20.

The first attribute information and the second attribute information are not limited to the examples illustrated in Fig. 4. The first attribute information may be the "type of engraved mark", the "presence or absence of coating", or a "taking form", and may be information indicating an attribute understood objectively. The second attribute information may be "soft on the tongue" or "easy to hold". Even though an attribute influences a sense (for example, a taste) common to users, sensitivity words which may have different expressions among the users are included in the second attribute information.

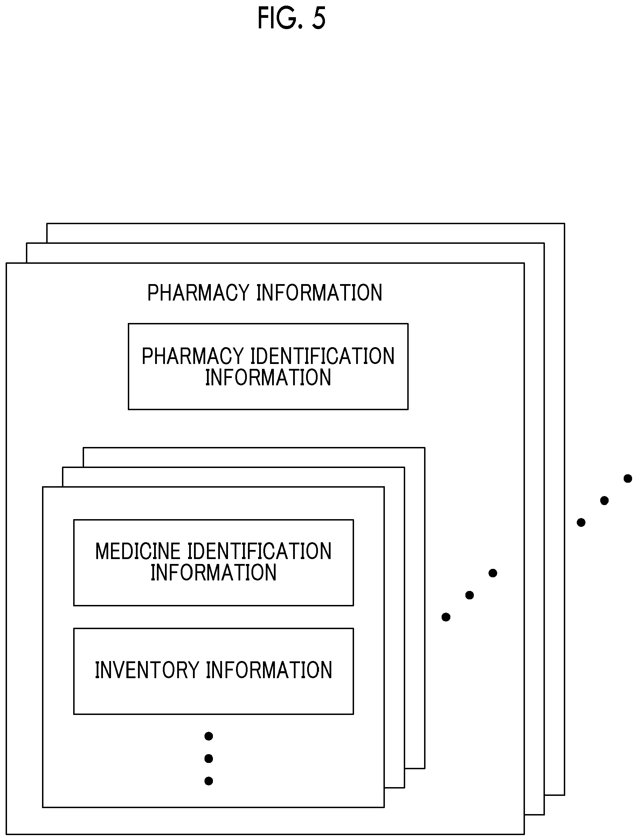

As illustrated in Fig. 5, the pharmacy information includes identification information (pharmacy identification information) for identifying a pharmacy, identification information of a medicine (hereinafter, referred to as a "handled medicine") handled in the pharmacy, and inventory information regarding an inventory of handled medicines.

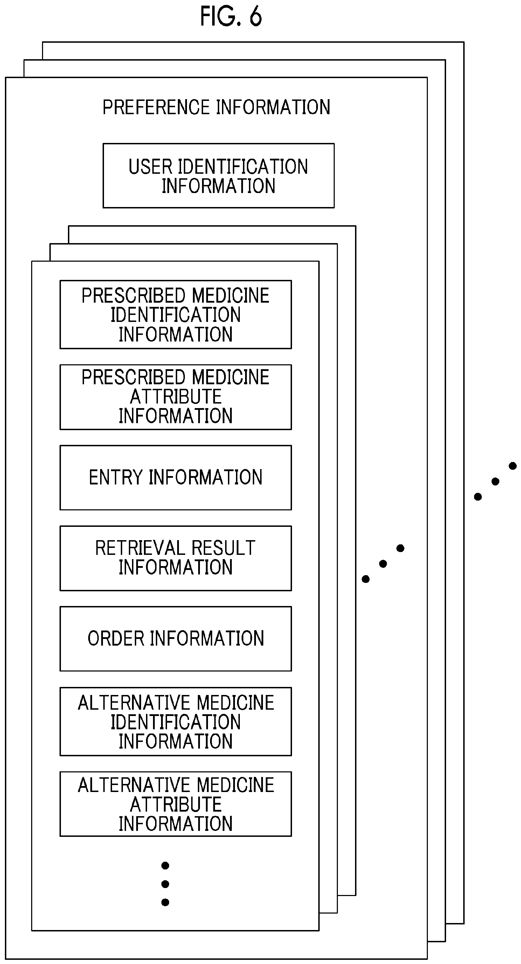

The preference information is information indicating a user's preference regarding attribute information of a medicine. As illustrated in Fig. 6, the preference information in this example includes user identification information for identifying a user, identification information and attribute information of a prescribed medicine, entry information which is preference information entered by the user in the user terminal 10, retrieval result information indicating a retrieval result of an alternative medicine, ordering information indicating a content of the user ordering a medicine, and identification information and attribute information of a medicine (including an alternative medicine) inspected by the inspection apparatus 20. The preference information (preference information regarding attribute information of a medicine) entered by the user is correlated with replacement of a prescribed medicine with an alternative medicine, and is stored in the database 30.

The preference information (for example, preference information entered by a user) includes at least one of first preference information for the first attribute information understood objectively or second preference information for the second attribute information understood subjectively by the user. The retrieval result information regarding preference information includes at least one of retrieval result information with the first preference information as a retrieval key or retrieval result information with the second preference information as a retrieval key.

Each of the server apparatus 40 and the administrator terminal 80 is configured with a computer apparatus. The database 30 is configured with, for example, a computer apparatus.

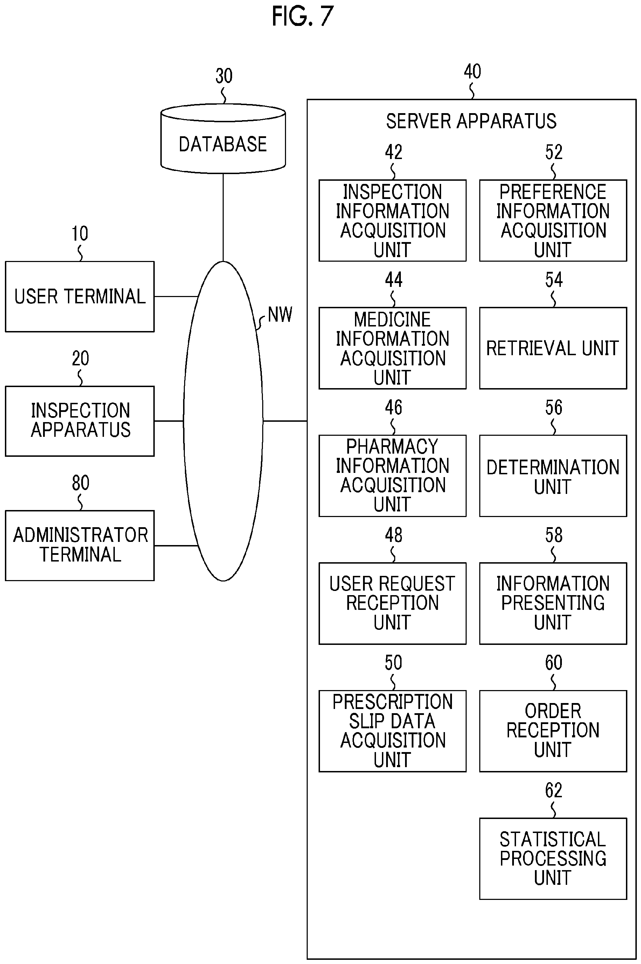

Fig. 7 is a block diagram illustrating a configuration example of the server apparatus in the first embodiment.

The server apparatus 40 comprises an inspection information acquisition unit 42 which acquires medicine inspection information in a pharmacy; a medicine information acquisition unit 44 which acquires medicine information; a pharmacy information acquisition unit 46 which acquires pharmacy information; a user request reception unit 48 which receives a user's request; a prescription slip data acquisition unit 50 which acquires prescription slip data of a user; a preference information acquisition unit 52 which acquires preference information of a user regarding attribute information of a medicine; a retrieval unit 54 which retrieves an alternative medicine which can replace a prescribed medicine designated in prescription slip data; a determination unit 56 which determines the suitability of an alternative medicine for preference information of a user; an information presenting unit 58 which presents a retrieval result to a user; an order reception unit 60 which receives an order for medicine purchase from a user; and a statistical processing unit 62 which performs a statistical process.

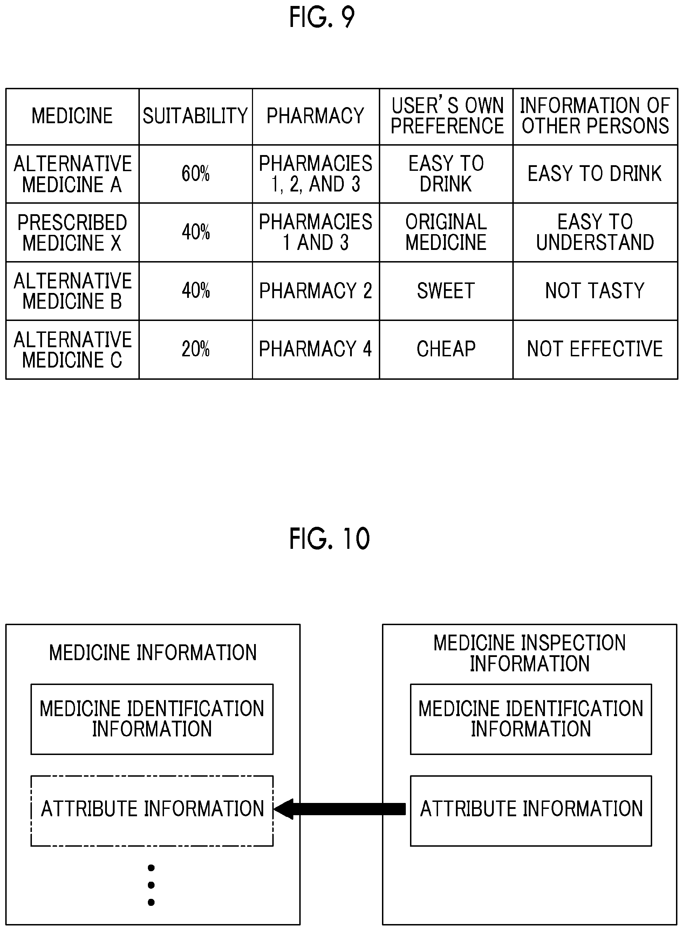

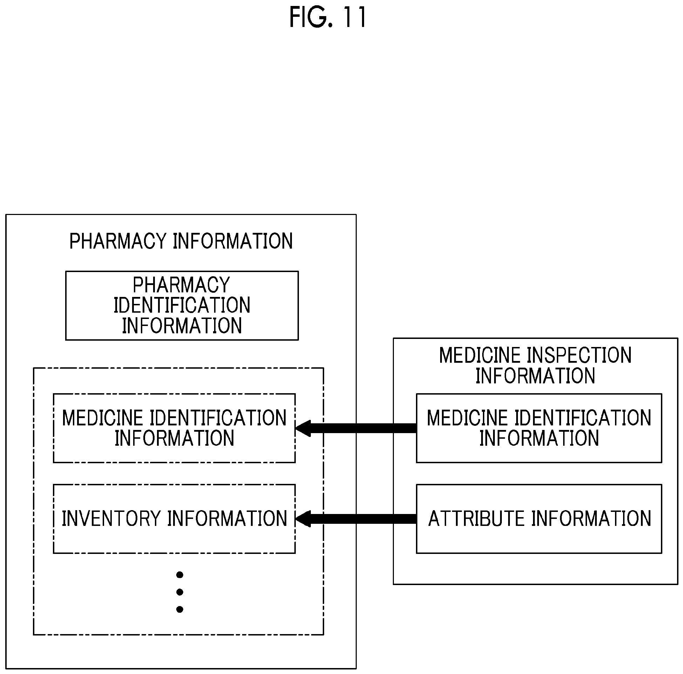

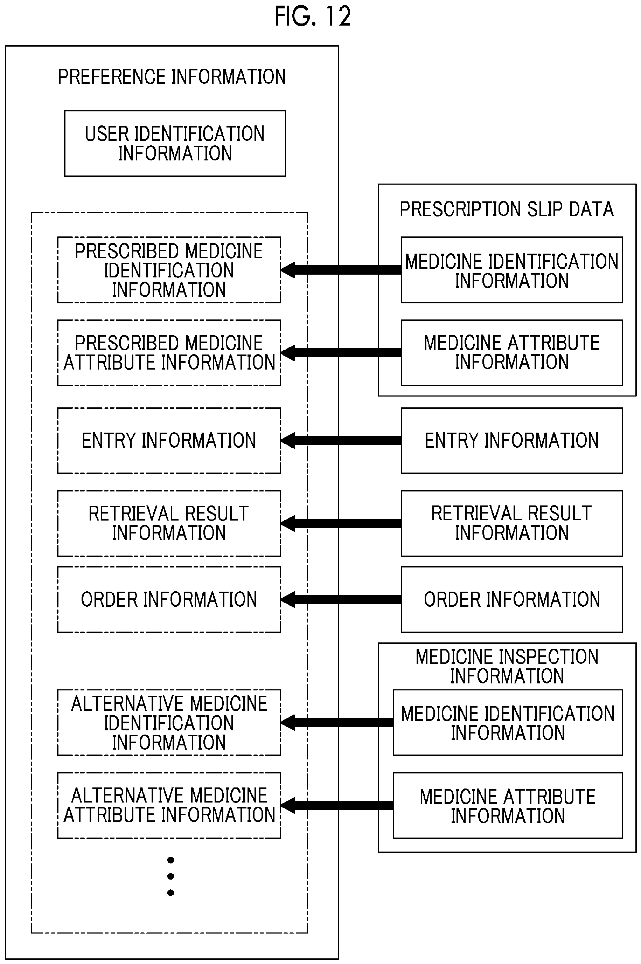

The inspection information acquisition unit 42 is configured with a communication device, and collects medicine inspection information of each pharmacy from a plurality of inspection apparatuses 20 respectively disposed in a plurality of pharmacies via the network NW. The medicine inspection information is generated through an inspection of a medicine in a pharmacy, using the inspection apparatus 20. As will be described later with reference to Fig. 10, the medicine inspection information includes at least medicine identification information and medicine attribute information. As will be described later with reference to Fig. 11, the medicine inspection information may further include part of pharmacy information, and include information which is a source of the pharmacy information. As will be described later with reference to Fig. 12, the medicine inspection information may further include part of preference information, and include information which is a source of the preference information. The inspection information acquisition unit 42 may acquire an image of a medicine obtained by imaging the medicine in the inspection apparatus 20 as the medicine inspection information. In other words, the inspection information acquisition unit 42 may acquire the medicine inspection information including the image of the medicine obtained by imaging the medicine in the inspection apparatus 20.

The medicine information acquisition unit 44 is configured with a central processing unit (CPU), acquires medicine attribute information from the medicine inspection information in the pharmacy, and causes the database 30 to update attribute information of medicine information. The medicine information acquisition unit 44 may acquire the medicine attribute information on the basis of an image of a medicine through image processing.

The pharmacy information acquisition unit 46 is configured with a CPU, acquires pharmacy information on the basis of medicine inspection information in a pharmacy, and causes the database 30 to update pharmacy information. The pharmacy information acquisition unit 46 may acquire the pharmacy information on the basis of an image of a medicine through image processing.

The user request reception unit 48 is configured with a communication device, and receives a request which is input in the user terminal 10 from the user terminal 10 via the network NW.

The prescription slip data acquisition unit 50 is configured to include a communication device, and receives a prescription slip image obtained by imaging a prescription slip in the user terminal 10 from the user terminal 10 via the network NW. The prescription slip data acquisition unit 50 is configured to include a CPU, and acquires prescription slip data on the basis of the prescription slip image through an optical character recognition (OCR) process.

The preference information acquisition unit 52 is configured with a CPU, acquires a user's preference information regarding medicine attribute information, and causes the database 30 to update preference information.

The preference information acquisition unit 52 in this example may acquire preference information according to various methods.

First, a user is requested to present preference information, and acquires information presented by the user in response to the request as preference information. Information regarding a medicine which the user has experience of taking may be acquired as preference information. Second, preference information is acquired on the basis of retrieval result information corresponding to a retrieval request from the user. The retrieval result information includes a retrieval key, and information indicating whether or not the user pays attention to (checks) a retrieval result. Third, in a case where an order for medicine purchase is received from the user, and preference information is acquired on the basis of a content of the order, and is correlated with acquired preference information. Fourth, a correspondence relationship between a prescribed medicine and an alternative medicine, defined through an inspection in the inspection apparatus 20, is acquired from medicine inspection information as preference information, and is correlated with acquired preference information.

The retrieval unit 54 is configured with a CPU, and retrieves an alternative medicine by referring prescription slip data, medicine information, and preference information of a user in response to a retrieval request from the user. Medicine information (hereinafter, referred to as "alternative medicine information") of an alternative medicine which is a result of the retrieval includes at least alternative medicine identification information and alternative medicine attribute information as described with reference to Fig. 3. In other words, the retrieval unit 54 acquires alternative medicine information including at least alternative medicine identification information and alternative medicine attribute information as a retrieval result of the alternative medicine. The alternative medicine information may further include an image of the alternative medicine.

The determination unit 56 is configured with a CPU, and calculates, as the suitability, a ratio (the number of suitable items/a total number of items) of the number of items in which an alternative medicine is suitable (the number of suitable items) to the number of all items (a total number of items) indicating a user's preference.

The information presenting unit 58 presents alternative medicine information including a retrieval result and pharmacy information regarding a pharmacy handling a prescribed medicine and a pharmacy handling a retrieved alternative medicine, to a user via the user terminal 10. The information presenting unit 58 presents information (alternative medicine information) regarding the retrieved alternative medicine to the user via the user terminal 10 along with the suitability. The information presenting unit 58 in this example is configured to include a communication device, and transmits presentation information which is to be presented to the user, to the user terminal 10 via the network NW. The information presenting unit 58 in this example is configured to include a CPU, and generates presentation information.

The order reception unit 60 is configured with a communication device, transmits an order screen information to the user terminal 10 via the network NW, and receives a medicine purchase order information from the user terminal 10.

The statistical processing unit 62 is configured with a CPU, and performs a statistical process on preference information stored in the database 30, so as to generate statistical information to be provided to a manufacturer or a pharmacy of an alternative medicine.

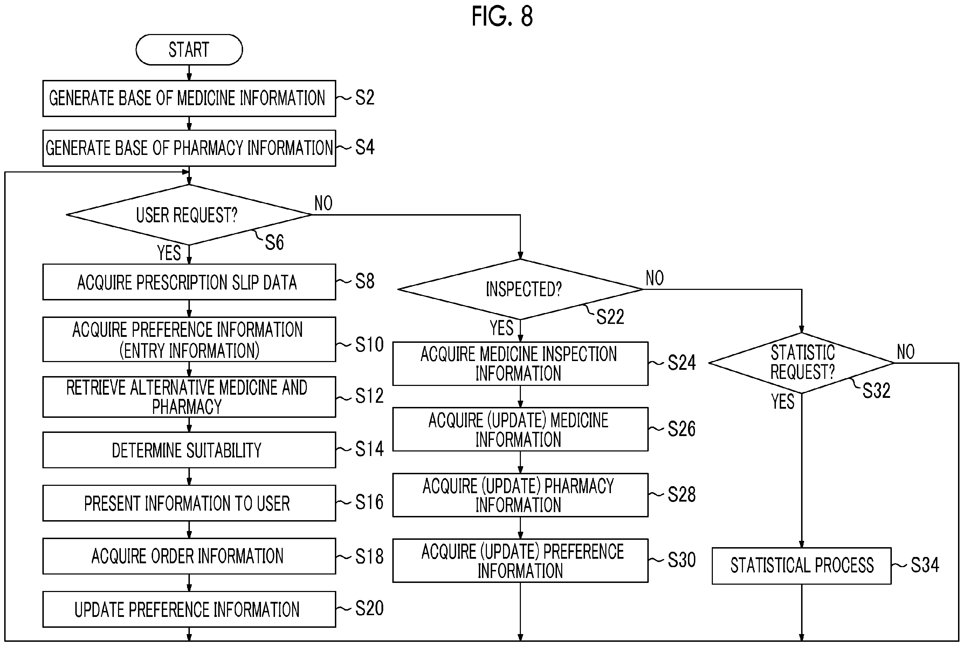

Fig. 8 is a flowchart illustrating a flow of an alternative medicine retrieval process example in the first embodiment. The alternative medicine retrieval process in this example is performed by the server apparatus 40 according to a program.

First, a medicine basic information which is a base of medicine information is generated in the database 30 by the medicine information acquisition unit 44 (step S2). The medicine basic information may be generated on the basis of, for example, a drug database available in the market. The medicine basic information includes at least medicine identification information. Medicine attribute information is updated by the medicine information acquisition unit 44 according to acquisition of medicine inspection information, and thus the medicine basic information may include minimum required attribute information. An ingredient amount (for example, XX mg) of a medicine (for example, a tablet) may be included in at least one of identification information or attribute information.

Next, pharmacy basic information which is a base of pharmacy information is generated in the database 30 by the pharmacy information acquisition unit 46 (step S4). The pharmacy basic information includes at least pharmacy identification information.

Next, it is determined whether or not a retrieval request is received from the user (step S6).

The user request reception unit 48 receives the user's alternative medicine retrieval request from the user terminal 10 via the network NW. The retrieval request in this example includes an instruction indicating whether a retrieval is performed on the basis of preference information of only the user's own (first user) having requested the retrieval or a retrieval is performed on the basis of preference information of other users who are different from the first user.

In a case where the user's retrieval request is received (YES in step S6), steps S8 to S20 are executed.

Prescription slip data of the user is acquired by the prescription slip data acquisition unit 50 from the user terminal 10 via the network NW (step S8; prescription slip data acquisition process).

The user terminal 10 in this example images the prescription slip Ps of the first user, and transmits an image (hereinafter, referred to as a "prescription slip image") of the prescription slip Ps obtained through the imaging to the server apparatus 40. The prescription slip data acquisition unit 50 performs an optical character recognition (OCR) process on the received prescription slip image so as to acquire prescription slip data with an electronic form. Prescription slip data which is manually entered on the user terminal 10 by the user may be acquired.

Next, the user's preference information regarding medicine attribute information is acquired by the preference information acquisition unit 52 from the user terminal 10 via the network NW (step S10; a preference information acquisition process).

The preference information acquired in this step is preference information of the first user which is entered on the user terminal 10. The preference information acquisition unit 52 in this example transmits a preference information entry request to the user terminal 10 via the network NW. In a case where the preference information entry request is received, the user terminal 10 requests the user to enter (present) the preference information. In a case where entry of the preference information is received from the user, the user terminal 10 transmits the user's preference information to the server apparatus 40 via the network NW. In other words, the preference information acquisition unit 52 controls the user terminal 10 via the network NW so as to request the user to present the preference information, and acquires, as the preference information, information presented from the user in response to the request.

For example, a medicine taken by the user (a medicine which the first user has experience of taking) is imaged by the user terminal 10, and an image of the taken medicine is transmitted to the server apparatus 40 from the user terminal 10 as the user's preference information. For example, as illustrated in Fig. 1, an image of one-dose package drugs OPt which are currently taken or sheet package drugs SPt which are currently taken is transmitted from the user terminal 10. The preference information acquisition unit 52 may specify a manufacturer (pharmaceutical manufacturer) manufacturing the taken medicine through image processing on the received image, and may use identification information of the manufacturer as preference information. For example, in a case where identification information (also referred to as a "company code") of a manufacturer of a medicine is included in identification information engraved or printed on the medicine, the identification information of the manufacturer is recognized through an OCR process. In a case where identification information of a manufacturer of a medicine is not engraved or printed on the medicine, the identification information of the manufacturer may be obtained on the basis of identification information (also referred to as a "product code") of the medicine engraved or printed on the medicine. The acquired identification information of the manufacturer may be handled as the preference information of the user.

A questionnaire may be displayed in the user terminal 10, and a response to the questionnaire which is entered on the user terminal 10 may be acquired as the preference information. The preference information may be estimated on the basis of a medication history of the user which is entered as a response. Information regarding a medicine or a manufacturer regarded to be effective by the user may be acquired as the preference information.

Next, the retrieval unit 54 retrieves another medicine (alternative medicine) which can replace a prescribed medicine designated in the prescription slip data and a pharmacy which can handle the alternative medicine (step S12; a retrieval process). The retrieval unit 54 in this example retrieves an alternative medicine by referring to the prescription slip data acquired in step S8, the medicine information stored in the database 30, and the preference information acquired in step S10.

The retrieval unit 54 may perform an objective retrieval of retrieving an alternative medicine on the basis of objective preference information (first preference information) corresponding to attribute information (first attribute information) understood objectively, and a subjective retrieval of retrieving an alternative medicine on the basis of subjective preference information (second preference information) corresponding to attribute information (second attribute information) understood subjectively by the user. In the subjective retrieval, in a case where conversion information (the correspondence relationship CR in Fig. 4) is stored in the database 30, subjective preference information is converted into objective preference information on the basis of the conversion information, and an alternative medicine is retrieved in the objective space OS with the objective preference information as a retrieval key. However, in a case where the second attribute information is correlated with medicine identification information, the retrieval unit 54 may retrieve an alternative medicine with subjective preference information as a retrieval key.

The retrieval unit 54 in this example may retrieve an alternative medicine by referring to preference information of other users who are different from the user (first user) having requested the retrieval. For example, a medicine of a manufacturer regarded to be effective by other users may be retrieved.

Next, the suitability of the alternative medicine for the preference information of the user is determined by the determination unit 56 (step S14; a suitability determination process). For example, a ratio (the number of suitable items/a total number of items) of the number of items in which a retrieved alternative medicine is suitable (the number of suitable items) to the number of all items (a total number of items) indicating the preference of the first user having requested the retrieval is calculated as the suitability. Calculation of the suitability in this example corresponds to "determination of the suitability" in the present invention.

Next, the information presenting unit 58 presents, to the user, medicine information regarding the prescribed medicine and medicine information regarding the retrieved alternative medicine (alternative medicine information), and pharmacy information regarding a pharmacy handling the prescribed medicine and a pharmacy handling the retrieved alternative medicine (step S16; an information presentation process). Fig. 9 illustrates an example in which the name of a prescribed medicine (a "prescribed medicine X") and the names of alternative medicines (an "alternative medicine A", an "alternative medicine B", and an "alternative medicine C"), and pharmacies having inventories of respective medicines among a plurality of pharmacies (a "pharmacy 1", a "pharmacy 2", a "pharmacy 3" and a "pharmacy 4") are displayed to the user on the user terminal 10. Medicine attribute information and pharmacy fixed information (an address, a telephone number, or the like) may further be displayed.

The information presenting unit 58 in this example may present, to the first user, the information (alternative medicine information) regarding the alternative medicine retrieved in the retrieval process (step S12) along with the suitability determined in the suitability determination process (step S14) via the user terminal 10. Fig. 9 illustrates an example in which the suitability (the "suitability" of each medicine) calculated by the determination unit 56 in step S14 is displayed.

The information presenting unit 58 may present preference information of other users who are different from the first user to the first user via the user terminal 10. In Fig. 9, "user's own preference" is preference information of the first user, and "information of other persons" is preference information of other users. In Fig. 9, for convenience of illustration, only a single piece of representative preference information is illustrated for each medicine, but this is only an example. For example, in a case where five items (for example, "easy to drink", an "original medicine", "sweet", "cheap", and "easy to understand") is designated as preferences of the first user by the first user, among suitable items for the "alternative medicine A", an example is illustrated in which only a suitable item (for example, "easy to drink") with the highest priority for the first user is displayed in the "user's own preference" field of the "alternative medicine A", but other suitable items (for example, "sweet" and "easy to understand") may be displayed. An example is illustrated in which only an evaluation item (for example, "easy to drink") receiving the most evaluations from other users for the "alternative medicine A" is displayed in the "information of other persons" field of the "alternative medicine A", but other evaluation items may be displayed. In a case where the preference information of the first user is different from the preference information of other users, the information presenting unit 58 may prioritize the preference information of the first user to the preference information of other users, and may present alternative medicine information. For example, with respect to a medicine for which the first user answered "effective" in the past questionnaire, even in a case where another user answers "not effective" in the identical questionnaire, the answer of the first user may be prioritized, and the suitability of a medicine may be calculated and displayed.

Next, the order reception unit 60 receives an order for medicine purchase from the first user corresponding to the retrieval result, from the user terminal 10 via the network NW, and thus order information is acquired (step S18; an order information acquisition process). However, in a case where there is no order reception, step S18 is skipped.

Next, the database 30 updates the preference information of the first user according to the prescription slip data acquired in step S8, the entry information acquired in step S10, the retrieval result information in step S12, and the order information (a content of the order of the user) acquired in step S18 (step S20; a preference information update process).

In a case where an inspection target medicine (OPa or SPa in Fig. 1) is inspected by the inspection apparatus 20, in the server apparatus 40, the inspection information acquisition unit 42 receives an inspection notification from the inspection apparatus 20 via the network NW.

It is determined whether or not the inspection notification is received from the inspection apparatus 20 (step S22), and, in a case where the inspection notification is received (YES in step S22), steps S24 to S30 are executed.

The inspection information acquisition unit 42 acquires medicine inspection information from the inspection apparatus 20 via the network NW (step S24; an inspection information acquisition process).

Next, the medicine information acquisition unit 44 acquires medicine information (step S26; a medicine information acquisition process). The acquired medicine information is stored in the database 30.

In a case where the inspection information acquisition unit 42 acquires the medicine inspection information, the medicine information acquisition unit 44 acquires medicine information from the medicine inspection information as illustrated in Fig. 10, and updates medicine information in the database 30. In other words, the database 30 (an aspect of a "medicine information storage unit") updates medicine attribute information according to the acquisition of the medicine inspection information. In a case where identification information of the inspection target medicine has been registered in the database 30, the registered identification information is correlated with the attribute information extracted from the medicine inspection information. In a case where the identification information of the inspection target medicine is not registered in the database 30, identification information and attribute information of the inspection target medicine acquired from the medicine inspection information may be registered in the database 30.

In a case where an image of the inspection target medicine is included in the medicine inspection information acquired from the inspection apparatus 20, the medicine information acquisition unit 44 in this example acquires attribute information of the medicine on the basis of the image of the medicine. In other words, the database 30 updates the attribute information of the medicine on the basis of the image of the medicine.

The pharmacy information acquisition unit 46 acquires pharmacy information (step S28; a pharmacy information acquisition process). The acquired pharmacy information is stored in the database 30.

In a case where the medicine inspection information is acquired by the inspection information acquisition unit 42, as illustrated in Fig. 11, the pharmacy information acquisition unit 46 acquires pharmacy information on the basis of the medicine inspection information, and updates the pharmacy information in the database 30. In other words, the database 30 (an aspect of a "pharmacy information storage unit") updates the pharmacy information according to the acquisition of the medicine inspection information. In a case where the alternative medicine is inspected by the inspection apparatus 20, identification information of the alternative medicine is acquired as identification information of a medicine handled in a pharmacy. A quantity of inspected medicines is included in the medicine inspection information as attribute information, and is acquired as change information of inventory information regarding a medicine handled in a pharmacy.

In a case where an image of the inspection target medicine is included in the medicine inspection information acquired from the inspection apparatus 20, the pharmacy information acquisition unit 46 in this example acquires pharmacy information on the basis of the image of the medicine. In other words, the database 30 updates the pharmacy information on the basis of the image of the medicine.

Next, the preference information acquisition unit 52 acquires preference information of the user (step S30; a preference information acquisition process). The acquired preference information is stored in the database 30.

As illustrated in Fig. 12, identification information and attribute information of the prescribed medicine are acquired from the prescription slip data acquired in step S8, the entry information (preference information entered by the user) is acquired in step S10, the retrieval result information is acquired in step S12, the order information is acquired in step S18, and the identification information and the attribute information of the alternative medicine are acquired from the medicine inspection information in step S26.

In step S30, the identification information and the attribute information of the alternative medicine acquired from the medicine inspection information are correlated with the identification information of the prescribed medicine and the preference information acquired in step S10 or the like, and are stored in the database 30. In other words, the database 30 (an aspect of a "preference information storage unit") stores the preference information in correlation with replacement of the prescribed medicine with the alternative medicine. In a case where the medicine inspection information is acquired by the inspection information acquisition unit 42, as illustrated in Fig. 12, the preference information acquisition unit 52 in this example acquires preference information on the basis of the medicine inspection information, and updates the preference information in the database 30. In other words, the database 30 (an aspect of a "preference information storage unit") updates the preference information of the user according to the acquisition of the medicine inspection information.

The server apparatus 40 in this example receives a statistical request from the administrator terminal 80 via the network NW. A statistical request may be generated in the server apparatus 40 every predetermined period.

It is determined whether or not a statistical request is received (step S32), and, in a case where the statistical request is received (YES in step S32), step S34 is executed.

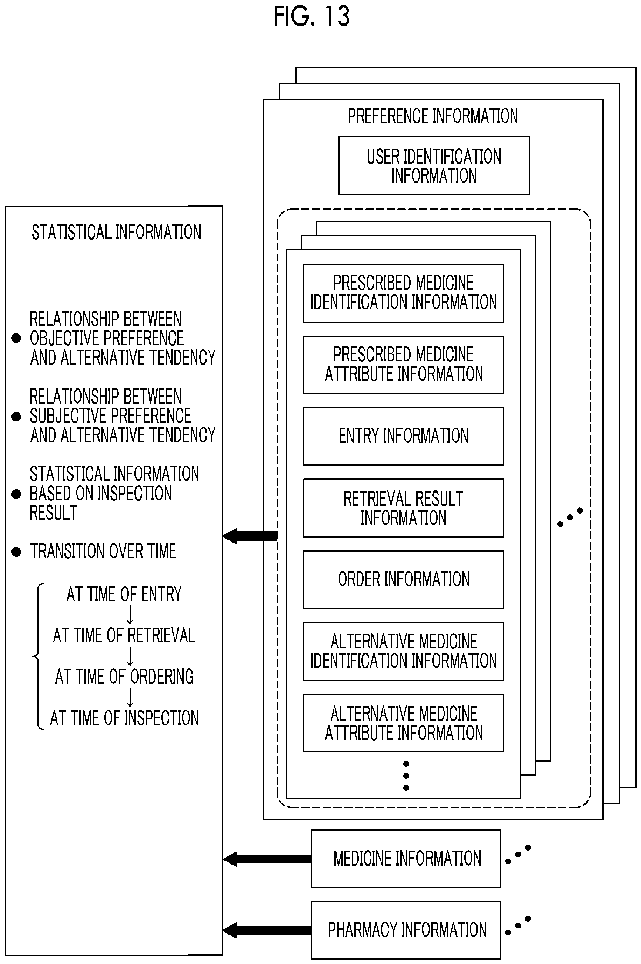

As illustrated in Fig. 13, the statistical processing unit 62 generates statistical information on the basis of the identification information and the attribute information of the prescribed medicine, the entry information of the user, the retrieval result information, the order information, and the identification information and the attribute information of the alternative medicine. In other words, the statistical processing unit 62 performs a statistical process on the information stored in the database 30 so as to generate statistical information. In user information, information (user identification information, for example, a personal name of the user) which can specify the individual user is excluded, and is not included in the statistical information. In user information, useful information such as a sex or an age group which does not specify the individual user is preferably included in the statistical information.

The statistical information generated by the statistical processing unit 62 has the following usefulness.

First, it is possible to understand a correspondence relationship between a user's subjective preference and an alternative tendency as a tendency to replace a prescribed medicine with an alternative medicine. This is because subjective preference information corresponding to the second attribute information understood subjectively by a user is included in the user's entry information and retrieval result information acquired by the preference information acquisition unit 52.

Second, it is possible to obtain statistical information with high reliability based on an inspection as a tendency to replace a prescribed medicine with an alternative medicine.

Third, it is possible to obtain statistical information reflecting therein transition over time from entry of preference information of a user to an inspection of an alternative medicine through a retrieval of the alternative medicine.

### [Second Embodiment]

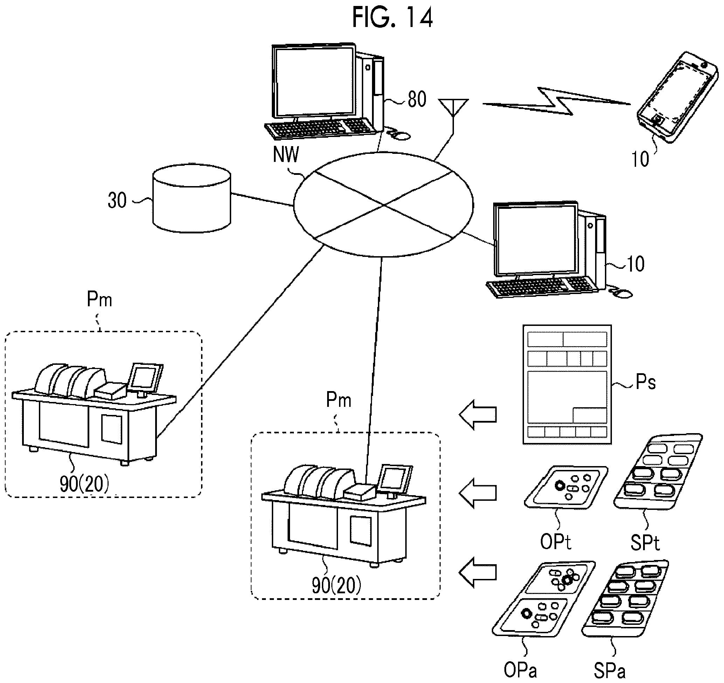

Fig. 14 is a system configuration diagram illustrating an example of a configuration of an alternative medicine retrieval system in a second embodiment. In Fig. 14, the same constituent elements as those in the alternative medicine retrieval system of the first embodiment illustrated in Fig. 1 are given the same reference numerals.

In the second embodiment, an alternative medicine retrieval apparatus 70 is configured with a pharmacy apparatus 90 disposed in each of a plurality of pharmacies Pm and a database 30. The pharmacy apparatus 90 in this example includes the inspection apparatus 20 illustrated in Fig. 1.

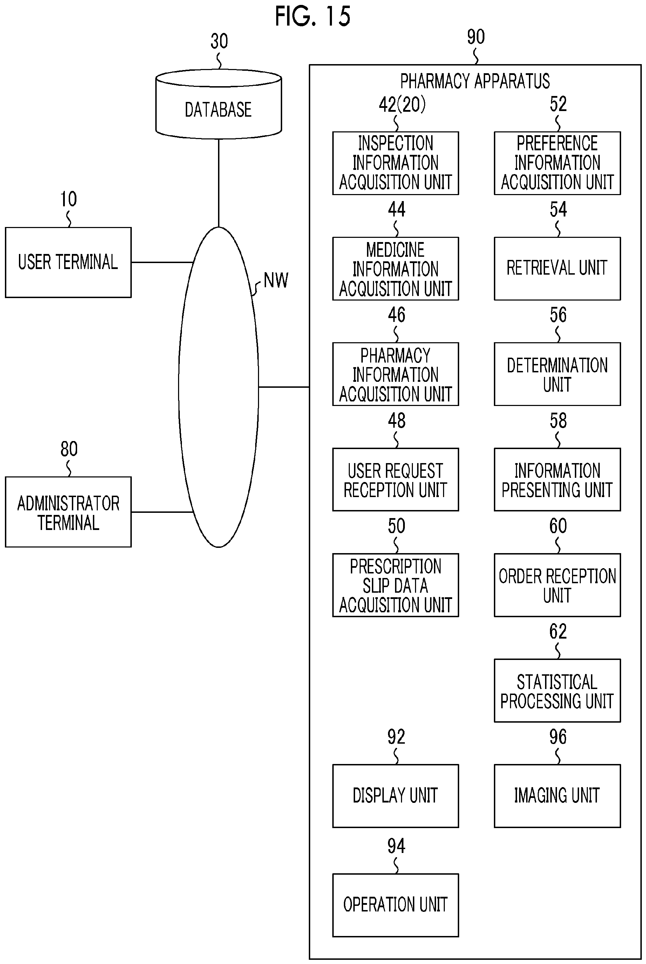

Fig. 15 is a block diagram illustrating a configuration example of the pharmacy apparatus 90 in the second embodiment. In Fig. 15, the same constituent elements as those of the server apparatus 40 of the first embodiment illustrated in Fig. 7 are given the same reference numerals. The inspection information acquisition unit 42 in this example has the same function as that of the inspection apparatus 20 of the first embodiment. The pharmacy apparatus 90 comprises a display unit 92 which performs display to a user, an operation unit 94 operated by a pharmacy member or the user, and an imaging unit 96 which images a prescription slip or a medicine.

In the present embodiment, the user goes to the pharmacy Pm, and orders an alternative medicine. The user may present medicines OPt and SPt which are currently taken at the pharmacy Pm.

There may be a configuration in which some of the constituent elements of the pharmacy apparatus 90 may be provided in a server apparatus configured with a computer apparatus, the server apparatus may be connected to the network NW. For example, the statistical processing unit 62 may be provided in the server apparatus.

### [Compliance with Laws and Regulations]

Although the embodiments for carrying out the present invention have been described, it is necessary to comply with laws and standards in a country or region in which the present invention is implemented, and it may be necessary to partially restrict or change the above-mentioned implementation details. For example, in a case where medicine mail order sales are not authorized, it is necessary to implement the present invention through restriction to an aspect of purchasing medicines at a pharmacy. It is necessary to pay sufficient attention such that a user can be checked by a doctor and a pharmacist as necessary.

In terms of security, it is necessary to pay sufficient attention to handling user personal information, and it is necessary to minimize collection of user personal information and presentation to others.

To summarize, the present invention may be implemented with sufficient attention to laws, safety, and security while achieving a user's convenience.

### [Program causing Computer to Function as Alternative Medicine Retrieval Apparatus]

A program causing a computer apparatus to realize the function (referred to as an "alternative medicine retrieval function") of the alternative medicine retrieval apparatus described in the embodiments may be stored in a non-transitory information storage medium which is a corporeal thing, and the program may be provided via the information storage medium. Examples of non-transitory information storage media may be various storage media such as a compact disk-read only memory (CD-ROM), a magnetic disk, and a memory card.

The program causes, for example, one or a plurality of computer apparatuses to function as an alternative medicine retrieval apparatus configured with the database 30 and the server apparatus 40 illustrated in Fig. 1. The program causes, for example, one or a plurality of computer apparatuses to function as an alternative medicine retrieval apparatus configured with the database 30 and the pharmacy apparatus 90 illustrated in Fig. 15.

The present invention is not limited to the adjustment embodiments and modification examples, and may be variously modified within the scope without departing from the spirit of the present invention.

### Explanation of References

10: user terminal
20: inspection apparatus
30: database
40: server apparatus
42: inspection information acquisition unit
44: medicine information acquisition unit
46: pharmacy information acquisition unit
48: user request reception unit
50: prescription slip data acquisition unit
52: preference information acquisition unit
54: retrieval unit
56: determination unit
58: information presenting unit
60: order reception unit
62: statistical processing unit
70: alternative medicine retrieval apparatus
80: administrator terminal
90: pharmacy apparatus
92: display unit
94: operation unit
96: imaging unit
CR: correspondence relationship (conversion information)
NW: network
OPa and OPt: one-dose package drugs
OS: objective space
Pm: pharmacy
Ps: prescription slip
Spa and SPt: sheet package drugs
SS: subjective space

## Claims

1. An alternative medicine retrieval apparatus comprising:
a medicine information storage unit that stores medicine information including identification information of a medicine, attribute information of the medicine, and identification information of a medicine which is able to replace the medicine;
a preference information acquisition unit that acquires preference information of a user regarding the attribute information;
a prescription slip data acquisition unit that acquires prescription slip data of a first user;
a pharmacy information acquisition unit that acquires pharmacy information including information regarding a medicine handled in a pharmacy and inventory information of the handled medicine;
a retrieval unit that retrieves an alternative medicine which is able to replace a prescribed medicine designated in the prescription slip data, and retrieves the alternative medicine by referring to the prescription slip data, the medicine information, and preference information of the first user in response to a request from the first user;
an information presenting unit that presents alternative medicine information including a result of the retrieval, and the pharmacy information regarding a pharmacy handling the prescribed medicine and a pharmacy handling the retrieved alternative medicine, to the first user; and
an inspection information acquisition unit that acquires medicine inspection information in the pharmacies,
wherein the medicine information storage unit updates the attribute information according to acquisition of the medicine inspection information.

2. The alternative medicine retrieval apparatus according to claim 1, further comprising:
a pharmacy information storage unit that stores the pharmacy information,
wherein the pharmacy information storage unit updates the pharmacy information according to acquisition of the medicine inspection information.

3. The alternative medicine retrieval apparatus according to claim 1 or 2, further comprising:
a preference information storage unit that stores the preference information in correlation with replacement of the prescribed medicine with the alternative medicine; and
a statistical processing unit that performs a statistical process on information stored in the preference information storage unit so as to generate statistical information.

4. The alternative medicine retrieval apparatus according to claim 3,
wherein the preference information storage unit updates the preference information according to acquisition of the medicine inspection information.

5. The alternative medicine retrieval apparatus according to any one of claims 1 to 4,
wherein the retrieval unit retrieves the alternative medicine by also referring to preference information of users other than the first user.

6. The alternative medicine retrieval apparatus according to any one of claims 1 to 5,
wherein the alternative medicine information includes preference information of users other than the first user.

7. The alternative medicine retrieval apparatus according to any one of claims 1 to 6,
wherein, in a case where the preference information of the first user is different from preference information of users other than the first user, the information presenting unit prioritizes the preference information of the first user, and presents the alternative medicine information.

8. The alternative medicine retrieval apparatus according to any one of claims 1 to 7, further comprising:
a determination unit that determines a suitability of the alternative medicine for the preference information of the first user,
wherein the information presenting unit presents information regarding the retrieved alternative medicine to the first user along with the determined suitability.

9. The alternative medicine retrieval apparatus according to any one of claims 1 to 8,
wherein the inspection information acquisition unit acquires an image obtained by imaging the medicine as the medicine inspection information, and
wherein the medicine information storage unit updates the attribute information on the basis of the image.

10. The alternative medicine retrieval apparatus according to any one of claims 1 to 9,
wherein the attribute information includes first attribute information which is attribute information understood objectively and second attribute information which is attribute information understood subjectively by the user.

11. The alternative medicine retrieval apparatus according to claim 10,
wherein the preference information includes first preference information which is preference information corresponding to the first attribute information and second preference information which is preference information corresponding to the second attribute information.

12. The alternative medicine retrieval apparatus according to claim 11,
wherein the second attribute information is sensitivity words expressing sensitivity of a person corresponding to the first attribute information,
wherein the medicine information storage unit stores conversion information indicating a correspondence relationship between the first attribute information and the second attribute information, and
wherein, in a case where the second preference information is acquired by the preference information acquisition unit, the retrieval unit coverts the second preference information into the first preference information on the basis of the conversion information, and retrieves the alternative medicine on the basis of the first preference information.

13. The alternative medicine retrieval apparatus according to any one of claims 1 to 12,
wherein the preference information of the first user is updated according to a result of the retrieval.

14. The alternative medicine retrieval apparatus according to any one of claims 1 to 13, further comprising:
an order reception unit that receives an order for medicine purchase corresponding to a result of the retrieval from the first user,
wherein the preference information of the first user is updated according to a content of the order.

15. The alternative medicine retrieval apparatus according to any one of claims 1 to 14,
wherein the prescription slip data acquisition unit acquires the prescription slip data on the basis of a prescription slip image obtained by imaging a prescription slip.

16. The alternative medicine retrieval apparatus according to any one of claims 1 to 15,
wherein the preference information acquisition unit acquires information regarding a medicine which the user has experience of taking, as the preference information.

17. The alternative medicine retrieval apparatus according to any one of claims 1 to 16,
wherein the preference information acquisition unit requests the user to present the preference information, and acquires, as the preference information, information presented by the user in response to the request.

18. An alternative medicine retrieval method comprising:
a medicine information acquisition process of acquiring medicine information including identification information of a medicine, attribute information of the medicine, and identification information of a medicine which is able to replace the medicine;
a pharmacy information acquisition process of acquiring pharmacy information including information regarding a medicine handled in a pharmacy and inventory information of the handled medicine;
a preference information acquisition process of acquiring preference information of a user regarding the attribute information;
a prescription slip data acquisition process of acquiring prescription slip data of a first user;
a retrieval process of retrieving an alternative medicine which is able to replace a prescribed medicine designated in the prescription slip data, and retrieving the alternative medicine by referring to the prescription slip data, the medicine information, and preference information of the first user in response to a request from the first user;
an information presenting process of presenting alternative medicine information including a result of the retrieval, and the pharmacy information regarding a pharmacy handling the prescribed medicine and a pharmacy handling the retrieved alternative medicine, to the first user; and
an inspection information acquisition process of acquiring medicine inspection information in the pharmacies,
wherein, in the medicine information acquisition process, the attribute information is updated according to acquisition of the medicine inspection information.
